(19) 

Europäisches Patentamt

European Patent Office

Office européen des brevets

(11) **EP 4 522 267 B1**

(12) **EUROPEAN PATENT SPECIFICATION**

(45) Date of publication and mention
of the grant of the patent:
**17.09.2025 Bulletin 2025/38**

(21) Application number: **23751278.5**

(22) Date of filing: **27.07.2023**

(51) International Patent Classification (IPC):
*A61N 5/10* (2006.01)    *G16H 20/40* (2018.01)
*G16H 30/20* (2018.01)    *G16H 30/40* (2018.01)
*G16H 40/63* (2018.01)

(52) Cooperative Patent Classification (CPC):
**G16H 30/20; A61B 6/5223; A61N 5/1037;
A61N 5/1039; A61N 5/1049; G16H 20/40;
G16H 30/40; G16H 40/63**

(86) International application number:
**PCT/EP2023/070864**

(87) International publication number:
**WO 2025/021305 (30.01.2025 Gazette 2025/05)**

(54) **DETERMINING PHYSICALLY CORRECT DIGITALLY RECONSTRUCTED RADIOGRAPHS**

BESTIMMUNG DER PHYSISCH KORREKTEN DIGITAL REKONSTRUIERTEN RÖNTGENBILDER

DÉTERMINATION DE RADIOGRAPHIES RECONSTRUITES NUMÉRIQUEMENT ET
PHYSIQUEMENT CORRECTES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**19.03.2025 Bulletin 2025/12**

(73) Proprietor: **Brainlab AG
81829 München (DE)**

(72) Inventors:
• **WOHLGEMUTH, Richard
81829 München (DE)**

• **BERLINGER, Kajetan
81829 München (DE)**

(74) Representative: **SSM Sandmair
Patentanwälte Rechtsanwalt
Partnerschaft mbB
Joseph-Wild-Straße 20
81829 München (DE)**

(56) References cited:
**US-A1- 2022 305 290**

## Description

## FIELD OF THE INVENTION

The present invention relates to a computer-implemented method of determining imaging control data for controlling medical imaging for localizing the position of an anatomical body part, a corresponding computer program, a computer-readable storage medium storing such a program and a computer executing the program, as well as a medical system comprising an electronic data storage device and the aforementioned computer.

## TECHNICAL BACKGROUND

For imaging-based soft tissue localization (e.g. a lung tumour) there is a high demand to already simulate the X-ray images before the first treatment. The simulated X-ray images (DRRs) shall give an impression about detectability and trackability of the target in real X-ray images. The real X-ray images used for tracking during for example radiation treatment should allow reliably recognizing the target which is difficult if the target consists of soft tissue.

US 2022/0305290 A1 discloses a method of operating imaging and tracking. The method includes determining, for each angle of a plurality of angles from which tracking images can be generated by an imaging device, a value of a tracking quality metric for tracking a target based on an analysis of a projection generated at that angle. The method also includes selecting, by a processing device, a subset of the plurality of angles that have a tracking quality metric value that satisfies a tracking quality metric criterion, one or more angles of the subset to be used to generate a tracking image of the target during a treatment stage, wherein the subset comprises at least a first angle and a second angle that is at least separated by a minimum threshold from the first angle.

The present invention has the object of providing a method which allows for, for example supports, generation of appropriate X-ray images.

The present invention can be used for planning radiation treatment procedures e.g. in connection with a system for image-guided radiotherapy such as VERO® and ExacTrac Dynamic®, both products of Brainlab AG.

Aspects of the present invention, examples and exemplary steps and their embodiments are disclosed in the following. Different exemplary features of the invention can be combined in accordance with the invention wherever technically expedient and feasible.

## EXEMPLARY SHORT DESCRIPTION OF THE INVENTION

In the following, a short description of the specific features of the present invention is given which shall not be understood to limit the invention only to the features or a combination of the features described in this section.

The disclosed method encompasses generation of synthesized medical images from a tomographic planning image at an image contrast which allows to appropriately recognize a representation of soft tissue in the synthesized medical images. An imaging parameter, for example the energy, of a (virtual) beam of imaging radiation is adjusted to achieve such a contrast. The determined value of the imaging parameter can then be used later for image-based tracking of the anatomical body part during a medical procedure such as radiation treatment. For example, too high energy values should be avoided because they would result in a comparably low contrast of image constituents representing soft tissue because only a small fraction of the energy would be absorbed by the soft tissue.

## GENERAL DESCRIPTION OF THE INVENTION

In this section, a description of the general features of the present invention is given for example by referring to possible embodiments of the invention.

In general, the invention reaches the aforementioned object by providing, in a first aspect, a computer-implemented medical method of determining imaging control data for controlling medical imaging for localizing the position of an anatomical body part. The method comprises executing, on at least one processor of at least one computer (for example at least one computer being part of a navigation system), the following exemplary steps which are executed by the at least one processor.

In a (for example first) exemplary step, planning image data is acquired. For example, the planning image data describes a three-dimensional digital planning medical image of the anatomical body part. For example, the planning medical image is a tomographic image such as a computed X-ray tomography, a magnetic resonance tomography or an ultrasound tomography. For example, the anatomical body part comprises or consists of soft tissue. For example, the anatomical body part is subject to a changing vital state. The term of vital state encompasses at least one of a physiological state and a pathological state of the anatomical body part. For example, the physiological state, for example the size and/or position of the anatomical body part, changes due to a vital movement of the patient to whom the anatomical body part belongs. A vital movement is for example at least one of a breathing motion which influences the size and position of the lung as the anatomical body part, and a heartbeat which influences the size and position of the heart as the anatomical body part. The size and/or position of the anatomical body part may also be influenced by a state of the pathological state of the anatomical body part, for example due to a tumour growing or shrinking (for example, by radiation treatment or surgery) in or close to the anatomical body part. For example, the tumour development may increase or reduce the size of the anatomical body part and therefore positions of its constituents.

In a (for example second) exemplary step, synthetic imaging parameter data is acquired. For example, the synthetic imaging parameter describes at least one synthetic imaging parameter, for example a value of at least one synthetic imaging parameter, usable for determining a digital synthetic medical image of the anatomical body part based on the planning image data.

In a (for example third) exemplary step, synthetic image data is determined based on the planning image data and the synthetic imaging parameter data. For example, the synthetic image data describes a digital synthesized two-dimensional medical image (for example, a digitally reconstructed radiograph) of at least part of the anatomical body part. For example, the synthesized two-dimensional medical image is generated using a predetermined (for example, known) imaging geometry for the synthesizing.

For example, the synthesized two-dimensional medical image is generated from the planning medical image using the synthetic imaging parameter. For example, the synthetic image data is determined by applying the Beer-Lambert law to the planning image data. For example, the absorption coefficients or material density values for the imaging beam (i.e. beam of imaging radiation, for example virtual beam of imaging radiation) associated with the anatomical body part and for example anatomical structures surrounding the anatomical body part, i.e. the anatomy through which the (virtual) beam of imaging radiation is configured to pass, are or have been deduced from the planning medical image. In an example, the method according to the first aspect comprises acquiring the parameters of a trained learning algorithm (for example, an artificial intelligence algorithm such as a machine learning algorithm, specifically a convolutional neural network) trained by inputting, to the learning algorithm, a plurality of three-dimensional planning images of the anatomical body of different patients and at least one two-dimensional radiography of the anatomical body part associated with each of the three-dimensional planning images and generated using known imaging parameters and for example having a known imaging geometry relative to the associated three-dimensional planning image, such that the learning algorithm is trained to establish a relation between the at least one two-dimensional radiography of the anatomical body part and synthesized two-dimensional medical images generated from the associated three-dimensional planning image. The synthetic image data is then generated by the trained learning algorithm by inputting the planning image data to the trained learning algorithm.

In a (for example fourth) exemplary step, image contrast data is acquired. For example, the image contrast date describes a predetermined criterion to be fulfilled by the contrast of the synthesized two-dimensional medical image. Contrast is the contradiction in luminance or colour that makes an object (or its representation in an image or display) distinguishable. For example, contrast is determined by the difference in the colour and brightness of the object and other objects within the same field of view. For example, the contrast of the synthesized two-dimensional medical image is the maximum contrast of the synthesized two-dimensional medical image which is the contrast ratio or dynamic range of the synthesized two-dimensional medical image. For example, the predetermined criterion is a threshold for the contrast of the synthesized two-dimensional digital medical image, for example a predetermined threshold or the output of an optimization step resulting from an optimization procedure for optimizing the at least one imaging parameter so that the contrast of the synthesized two-dimensional medical image fulfils the predetermined criterion.

In a (for example fifth) exemplary step, localization imaging parameter data is determined based on the synthetic imaging parameter data and the synthetic image data and the image contrast data. For example, the localization parameter data describes at least one localization imaging parameter, for example a value of at least one localization imaging parameter, to be used for generating a digital localization medical image of the anatomical body part for localizing the position of the anatomical body part. For example, the synthetic medical image is a synthetic X-ray image, for example a synthetic radiography such as a digitally reconstructed radiograph (DRR), and the localization medical image is an X-ray image, for example a radiography Within this disclosure, localizing the anatomical body part means determining the position of the anatomical body part and encompasses the determination of a changing position of the anatomical body part which changes over, i.e. a time-dependent position, for example change of the position, of the anatomical body part. Determining a time-dependent position of the anatomical body part is also called tracking the anatomical body part. For example, the localization parameter data is determined if the contrast of the synthesized two-dimensional medical image fulfils, for example has a predetermined relationship to (for example, is at least substantially equal to or lower or higher than), the predetermined criterion. For example, the synthetic image data is determined using the synthetic imaging parameter and the at least one localization imaging parameter corresponds, for example is at least substantially equal, to the synthetic imaging parameter.

In an example of the method according to the first aspect, the synthetic imaging parameter and the localization imaging parameter describe at least one of an energy of an imaging beam used for imaging the anatomical body part and an exposure time of the imaging.

In a further example of the method according to the first aspect, the three-dimensional digital planning medical image includes a plurality of three-dimensional digital planning medical images describing the anatomical body part in different vital states For example, the digital synthesized two-dimensional medical image includes a plurality of synthesized two-dimensional medical images of at least part of the anatomical body part associated with, for example uniquely assigned to each of, the plurality of three-dimensional digital planning medical images, and the at least one synthetic imaging parameter and the contrast of the image representation of the anatomical body part and anatomical structures surrounding the anatomical body part in the synthesized two-dimensional medical image are varied to optimize localization of the image representation the anatomical body part in each of the plurality of synthesized two-dimensional medical images, for example such that a trajectory, for example a time-dependent trajectory, of the image representation of the anatomical body part described by the plurality of synthesized two-dimensional medical images corresponds, for example is similar to a predetermined extent, to a trajectory, for example a time-dependent trajectory, of the image representation of the anatomical body part described by the plurality of three-dimensional digital planning medical images. The similarity can be determined by applying an image fusion algorithm which establishes a mapping such as an elastic fusion between corresponding image constituents in the image representation of the anatomical body part described by the plurality of synthesized two-dimensional medical images and the image representation of the anatomical body part described by the plurality of three-dimensional digital planning medical images. Thereby, the trackability of a search pattern image for searching for the image representation of the anatomical body part in the plurality of synthesized two-dimensional medical images is improved, for example optimized.

In a further example of the method according to the first aspect, the digital synthesized two-dimensional medical image includes at least two digital synthesized two-dimensional medical images of the at least part of the anatomical body part which are or have been generated using different values of the at least one synthetic imaging parameter (for example, different energy values) and the at least two digital synthesized two-dimensional medical images are weighted (for example, in a range from 0 to 1, including these values) and subtracted from each other and the result such as an optimized result of the subtraction is used for determining the localization imaging parameter data.

In a further example, the method according to the first aspect comprises determining, based on the localization imaging parameter data, control data for controlling a medical imaging device, for example an X-ray device such as two-dimensional X-ray device, to generate an image of the anatomical body part. For example, the control data are determined such that the medical imaging device is controlled to generate the image of the anatomical body part by using the localization imaging parameter. For example, the control data describes a control signal to be issued to the medical imaging device to generate an image of the anatomical body part using the localization imaging parameter. In an example, the method according to the first aspect comprises execution of the control data, for example to generate the image of the anatomical body part using the localization imaging parameter.

In a second aspect, the invention is directed to a computer program comprising instructions which, when the program is executed by at least one computer, causes the at least one computer to carry out method according to the first aspect. The invention may alternatively or additionally relate to a (physical, for example electrical, for example technically generated) signal wave, for example a digital signal wave, such as an electromagnetic carrier wave carrying information which represents the program, for example the aforementioned program, which for example comprises code means which are adapted to perform any or all of the steps of the method according to the first aspect. The signal wave is in one example a data carrier signal carrying the aforementioned computer program. A computer program stored on a disc is a data file, and when the file is read out and transmitted it becomes a data stream for example in the form of a (physical, for example electrical, for example technically generated) signal. The signal can be implemented as the signal wave, for example as the electromagnetic carrier wave which is described herein. For example, the signal, for example the signal wave is constituted to be transmitted via a computer network, for example LAN, WLAN, WAN, mobile network, for example the internet. For example, the signal, for example the signal wave, is constituted to be transmitted by optic or acoustic data transmission. The invention according to the second aspect therefore may alternatively or additionally relate to a data stream representative of the aforementioned program, i.e. comprising the program.

In a third aspect, the invention is directed to a computer-readable storage medium on which the program according to the second aspect is stored. The program storage medium is for example non-transitory.

In a fourth aspect, the invention is directed to at least one computer (for example, a computer), comprising at least one processor (for example, a processor), wherein the program according to the second aspect is executed by the processor, or wherein the at least one computer comprises the computer-readable storage medium according to the third aspect.

In a fifth aspect, the invention is directed to a medical system, comprising:

a) the at least one computer according to the fourth aspect, wherein the instructions cause the computer to carry out the method according to the first aspect as far as it includes determining the control data for controlling an medical imaging device, to generate an image of the anatomical body part;

b) at least one electronic data storage device storing the control data; and

c) a radiation treatment apparatus comprising at least one medical imaging device,

wherein the at least one computer is operably coupled to

- the at least one electronic data storage device for acquiring, from the at least one data storage device, the control data, and
- the radiation treatment apparatus for issuing a control signal to the radiation treatment apparatus for controlling the operation of the at least one medical imaging device on the basis of the control data.

The method according to the first aspect allows determination of a localization imaging parameter data to be used for tracking the anatomical body part during radiation treatment so that for example irradiation of the anatomical body part can, as desired, be avoided or effected by appropriately controlling emission of a treatment beam from the radiation treatment apparatus The localization imaging parameter is determined using the method according to the first aspect such that its value is suitable for imaging the anatomical body part if it comprises or consists of soft tissue and such that the imaging results in images of high contrast for soft tissue to be able to reliably determine its position from the resulting images. This is done for example by appropriately determining the energy of the imaging beam described by the localization imaging parameter. For example, too high energy values should be avoided because they would result in a comparably low contrast of image constituents representing soft tissue because only a small fraction of the energy would be absorbed by the soft tissue.

Alternatively or additionally, the invention according to the fifth aspect is directed to a for example non-transitory computer-readable program storage medium storing a program for causing the computer according to the fourth aspect to execute the data processing steps of the method according to the first aspect.

In an example of the system according to the fifth aspect, the medical device comprises a radiation treatment apparatus comprising a treatment beam source and a patient support unit (such as at least one of a patient bed or a headrest).

For example, the disclosed method is not a method for treatment of the human or animal body by surgery or therapy. For example, the invention does not involve or in particular comprise or encompass an invasive step which would represent a substantial physical interference with the body requiring professional medical expertise to be carried out and entailing a substantial health risk even when carried out with the required professional care and expertise.

For example, the invention does not comprise a step of radiation treatment. More particularly, the invention does not involve or in particular comprise or encompass any surgical or therapeutic activity. The invention is instead directed as applicable to determining an imaging parameter for imaging an anatomical body part. For this reason alone, no surgical or therapeutic activity and in particular no surgical or therapeutic step is necessitated or implied by carrying out the invention.

## DEFINITIONS

In this section, definitions for specific terminology used in this disclosure are offered which also form part of the present disclosure.

The method in accordance with the invention is for example a computer-implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term computer includes a server resource. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services™. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz®, a product of Brainlab AG. The monitor may also be the monitor of a portable, for example handheld, device such as a smart phone or personal digital assistant or digital media player.

The invention also relates to a computer program comprising instructions which, when on the program is executed by a computer, cause the computer to carry out the method or methods, for example, the steps of the method or methods, described herein and/or to a computer-readable storage medium (for example, a non-transitory computer-readable storage medium) on which the program is stored and/or to a computer comprising said program storage medium and/or to a (physical, for example electrical, for example technically generated) signal wave, for example a digital signal wave, such as an electromagnetic carrier wave carrying information which represents the program, for example the aforementioned program, which for example comprises code means which are adapted to perform any or all of the method steps described herein. The signal wave is in one example a data carrier signal carrying the aforementioned computer program. The invention also relates to a computer comprising at least one processor and/or the aforementioned computer-readable storage medium and for example a memory, wherein the program is executed by the processor.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer, a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

The expression "acquiring data" for example encompasses (within the framework of a computer implemented method) the scenario in which the data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. A step of "determining" as described herein for example comprises or consists of issuing a command to perform the determination described herein. For example, the step comprises or consists of issuing a command to cause a computer, for example a remote computer, for example a remote server, for example in the cloud, to perform the determination. Alternatively or additionally, a step of "determination" as described herein for example comprises or consists of receiving the data resulting from the determination described herein, for example receiving the resulting data from the remote computer, for example from that remote computer which has been caused to perform the determination. The meaning of "acquiring data" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

The information on the imaging geometry preferably comprises information which allows the analysis image (X-ray image) to be calculated, given a known relative position between the imaging geometry analysis apparatus and the analysis object (anatomical body part) to be analysed by X-ray radiation, if the analysis object which is to be analysed is known, wherein "known" means that the spatial geometry (size and shape) of the analysis object is known. This means for example that three-dimensional, "spatially resolved" information concerning the interaction between the analysis object (anatomical body part) and the analysis radiation (X-ray radiation) is known, wherein "interaction" means for example that the analysis radiation is blocked or partially or completely allowed to pass by the analysis object. The location and in particular orientation of the imaging geometry is for example defined by the position of the X-ray device, for example by the position of the X-ray source and the X-ray detector and/or for example by the position of the multiplicity (manifold) of X-ray beams which pass through the analysis object and are detected by the X-ray detector. The imaging geometry for example describes the position (i.e. the location and in particular the orientation) and the shape (for example, a conical shape exhibiting a specific angle of inclination) of said multiplicity (manifold). The position can for example be represented by the position of an X-ray beam which passes through the centre of said multiplicity or by the position of a geometric object (such as a truncated cone) which represents the multiplicity (manifold) of X-ray beams. Information concerning the above-mentioned interaction is preferably known in three dimensions, for example from a three-dimensional CT, and describes the interaction in a spatially resolved way for points and/or regions of the analysis object, for example for all of the points and/or regions of the analysis object. Knowledge of the imaging geometry for example allows the location of a source of the radiation (for example, an X-ray source) to be calculated relative to an image plane (for example, the plane of an X-ray detector). With respect to the connection between three-dimensional analysis objects and two-dimensional analysis images as defined by the imaging geometry, reference is made for example to the following publications:

1. "An Efficient and Accurate Camera Calibration Technique for 3D Machine Vision", Roger Y. Tsai, Proceedings of the IEEE Conference on Computer Vision and Pattern Recognition. Miami Beach, Florida, 1986, pages 364-374

2. "A Versatile Camera Calibration Technique for High-Accuracy 3D Machine Vision Metrology Using Off-the-Shelf TV Cameras and Lenses", Roger Y. Tsai, IEEE Journal of Robotics and Automation, Volume RA-3, No. 4, August 1987, pages 323-344.

3. Yaniv Z., Joskowicz L., Simkin A., Garza-Jinich M., Milgrom C. (1998) Fluoroscopic image processing for computer-aided orthopaedic surgery. In: Wells W.M., Colchester A., Delp S. (eds) Medical Image Computing and Computer-Assisted Intervention - MICCAI'98. MICCAI 1998. Lecture Notes in Computer Science, vol 1496. Springer, Berlin, Heidelberg

4. EP 08 156 293.6

5. US 61/054,187

Preferably, atlas data is acquired which describes (for example defines, more particularly represents and/or is) a general three-dimensional shape of the anatomical body part. The atlas data therefore represents an atlas of the anatomical body part. An atlas typically consists of a plurality of generic models of objects, wherein the generic models of the objects together form a complex structure. For example, the atlas constitutes a statistical model of a patient's body (for example, a part of the body) which has been generated from anatomic information gathered from a plurality of human bodies, for example from medical image data containing images of such human bodies. In principle, the atlas data therefore represents the result of a statistical analysis of such medical image data for a plurality of human bodies. This result can be output as an image - the atlas data therefore contains or is comparable to medical image data. Such a comparison can be carried out for example by applying an image fusion algorithm which conducts an image fusion between the atlas data and the medical image data. The result of the comparison can be a measure of similarity between the atlas data and the medical image data. The atlas data comprises image information (for example, positional image information) which can be matched (for example by applying an elastic or rigid image fusion algorithm) for example to image information (for example, positional image information) contained in medical image data so as to for example compare the atlas data to the medical image data in order to determine the position of anatomical structures in the medical image data which correspond to anatomical structures defined by the atlas data.

The human bodies, the anatomy of which serves as an input for generating the atlas data, advantageously share a common feature such as at least one of gender, age, ethnicity, body measurements (e.g. size and/or mass) and pathologic state. The anatomic information describes for example the anatomy of the human bodies and is extracted for example from medical image information about the human bodies. The atlas of a femur, for example, can comprise the head, the neck, the body, the greater trochanter, the lesser trochanter and the lower extremity as objects which together make up the complete structure. The atlas of a brain, for example, can comprise the telencephalon, the cerebellum, the diencephalon, the pons, the mesencephalon and the medulla as the objects which together make up the complex structure. One application of such an atlas is in the segmentation of medical images, in which the atlas is matched to medical image data, and the image data are compared with the matched atlas in order to assign a point (a pixel or voxel) of the image data to an object of the matched atlas, thereby segmenting the image data into objects.

For example, the atlas data includes information of the anatomical body part. This information is for example at least one of patient-specific, non-patient-specific, indication-specific or non-indication-specific. The atlas data therefore describes for example at least one of a patient-specific, non-patient-specific, indication-specific or non-indication-specific atlas. For example, the atlas data includes movement information indicating a degree of freedom of movement of the anatomical body part with respect to a given reference (e.g. another anatomical body part). For example, the atlas is a multimodal atlas which defines atlas information for a plurality of (i.e.

at least two) imaging modalities and contains a mapping between the atlas information in different imaging modalities (for example, a mapping between all of the modalities) so that the atlas can be used for transforming medical image information from its image depiction in a first imaging modality into its image depiction in a second imaging modality which is different from the first imaging modality or to compare (for example, match or register) images of different imaging modality with one another.

The present invention relates to the field of controlling a treatment beam. The treatment beam treats body parts which are to be treated and which are referred to in the following as "treatment body parts". These body parts are for example parts of a patient's body, i.e. anatomical body parts.

The present invention relates to the field of medicine and for example to the use of beams, such as radiation beams, to treat parts of a patient's body, which are therefore also referred to as treatment beams. A treatment beam treats body parts which are to be treated and which are referred to in the following as "treatment body parts". These body parts are for example parts of a patient's body, i.e. anatomical body parts. Ionising radiation is for example used for the purpose of treatment. For example, the treatment beam comprises or consists of ionising radiation. The ionising radiation comprises or consists of particles (for example, sub-atomic particles or ions) or electromagnetic waves which are energetic enough to detach electrons from atoms or molecules and so ionise them. Examples of such ionising radiation include X-rays, high-energy particles (high-energy particle beams) and/or ionising radiation emitted from a radioactive element. The treatment radiation, for example the treatment beam, is for example used in radiation therapy or radiotherapy, such as in the field of oncology. For treating cancer in particular, parts of the body comprising a pathological structure or tissue such as a tumour are treated using ionising radiation. The tumour is then an example of a treatment body part.

The treatment beam is preferably controlled such that it passes through the treatment body part. However, the treatment beam can have a negative effect on body parts outside the treatment body part. These body parts are referred to here as "outside body parts". Generally, a treatment beam has to pass through outside body parts in order to reach and so pass through the treatment body part.

Reference is also made in this respect to the following web pages: http://www.elekta.com/healthcare_us_elekta_vmat.php and http://www.varian.com/us/oncology/treatments/treatment_techniques/rapidarc.

A treatment body part can be treated by one or more treatment beams issued from one or more directions at one or more times. The treatment by means of the at least one treatment beam thus follows a particular spatial and temporal pattern. The term "beam arrangement" is then used to cover the spatial and temporal features of the treatment by means of the at least one treatment beam. The beam arrangement is an arrangement of at least one treatment beam.

The "beam positions" describe the positions of the treatment beams of the beam arrangement. The arrangement of beam positions is referred to as the positional arrangement. A beam position is preferably defined by the beam direction and additional information which allows a specific location, for example in three-dimensional space, to be assigned to the treatment beam, for example information about its co-ordinates in a defined co-ordinate system. The specific location is a point, preferably a point on a straight line. This line is then referred to as a "beam line" and extends in the beam direction, for example along the central axis of the treatment beam. The defined co-ordinate system is preferably defined relative to the treatment device or relative to at least a part of the patient's body. The positional arrangement comprises and for example consists of at least one beam position, for example a discrete set of beam positions (for example, two or more different beam positions), or a continuous multiplicity (manifold) of beam positions.

For example, one or more treatment beams adopt(s) the treatment beam position(s) defined by the positional arrangement simultaneously or sequentially during treatment (for example sequentially if there is only one beam source to emit a treatment beam). If there are several beam sources, it is also possible for at least a subset of the beam positions to be adopted simultaneously by treatment beams during the treatment. For example, one or more subsets of the treatment beams can adopt the beam positions of the positional arrangement in accordance with a predefined sequence. A subset of treatment beams comprises one or more treatment beams. The complete set of treatment beams which comprises one or more treatment beams which adopt(s) all the beam positions defined by the positional arrangement is then the beam arrangement.

In the field of medicine, imaging methods (also called imaging modalities and/or medical imaging modalities) are used to generate image data (for example, two-dimensional or three-dimensional image data) of anatomical structures (such as soft tissues, bones, organs, etc.) of the human body. The term "medical imaging methods" is understood to mean (advantageously apparatus-based) imaging methods (for example so-called medical imaging modalities and/or radiological imaging methods) such as for instance computed tomography (CT) and cone beam computed tomography (CBCT, such as volumetric CBCT), X-ray tomography, magnetic resonance tomography (MRT or MRI), conventional X-ray, sonography and/or ultrasound examinations, and positron emission tomography. For example, the medical imaging methods are performed by the analytical devices. Examples for medical imaging modalities applied by medical imaging methods are: X-ray radiography, magnetic resonance imaging, medical ultrasonography or ultrasound, endoscopy, elastography, tactile imaging, thermography, medical photography and nuclear medicine functional imaging techniques as positron emission tomography (PET) and Single-photon emission computed tomography (SPECT), as mentioned by Wikipedia. The image data thus generated is also termed "medical imaging data". Analytical devices for example are used to generate the image data in apparatus-based imaging methods. The imaging methods are for example used for medical diagnostics, to analyse the anatomical body in order to generate images which are described by the image data. The imaging methods are also for example used to detect pathological changes in the human body. However, some of the changes in the anatomical structure, such as the pathological changes in the structures (tissue), may not be detectable and for example may not be visible in the images generated by the imaging methods. A tumour represents an example of a change in an anatomical structure. If the tumour grows, it may then be said to represent an expanded anatomical structure. This expanded anatomical structure may not be detectable; for example, only a part of the expanded anatomical structure may be detectable. Primary/high-grade brain tumours are for example usually visible on MRI scans when contrast agents are used to infiltrate the tumour. MRI scans represent an example of an imaging method. In the case of MRI scans of such brain tumours, the signal enhancement in the MRI images (due to the contrast agents infiltrating the tumour) is considered to represent the solid tumour mass. Thus, the tumour is detectable and for example discernible in the image generated by the imaging method. In addition to these tumours, referred to as "enhancing" tumours, it is thought that approximately 10% of brain tumours are not discernible on a scan and are for example not visible to a user looking at the images generated by the imaging method.

Mapping describes a transformation (for example, linear transformation) of an element (for example, a pixel or voxel), for example the position of an element, of a first data set in a first coordinate system to an element (for example, a pixel or voxel), for example the position of an element, of a second data set in a second coordinate system (which may have a basis which is different from the basis of the first coordinate system). In one embodiment, the mapping is determined by comparing (for example, matching) the color values (for example grey values) of the respective elements by means of an elastic or rigid fusion algorithm. The mapping is embodied for example by a transformation matrix (such as a matrix defining an affine transformation).

Image fusion can be elastic image fusion or rigid image fusion. In the case of rigid image fusion, the relative position between the pixels of a 2D image and/or voxels of a 3D image is fixed, while in the case of elastic image fusion, the relative positions are allowed to change.

In this application, the term "image morphing" is also used as an alternative to the term "elastic image fusion", but with the same meaning.

Elastic fusion transformations (for example, elastic image fusion transformations) are for example designed to enable a seamless transition from one dataset (for example a first dataset such as for example a first image) to another dataset (for example a second dataset such as for example a second image). The transformation is for example designed such that one of the first and second datasets (images) is deformed, for example in such a way that corresponding structures (for example, corresponding image elements) are arranged at the same position as in the other of the first and second images. The deformed (transformed) image which is transformed from one of the first and second images is for example as similar as possible to the other of the first and second images. Preferably, (numerical) optimisation algorithms are applied in order to find the transformation which results in an optimum degree of similarity. The degree of similarity is preferably measured by way of a measure of similarity (also referred to in the following as a "similarity measure"). The parameters of the optimisation algorithm are for example vectors of a deformation field. These vectors are determined by the optimisation algorithm in such a way as to result in an optimum degree of similarity. Thus, the optimum degree of similarity represents a condition, for example a constraint, for the optimisation algorithm. The bases of the vectors lie for example at voxel positions of one of the first and second images which is to be transformed, and the tips of the vectors lie at the corresponding voxel positions in the transformed image. A plurality of these vectors is preferably provided, for instance more than twenty or a hundred or a thousand or ten thousand, etc. Preferably, there are (other) constraints on the transformation (deformation), for example in order to avoid pathological deformations (for instance, all the voxels being shifted to the same position by the transformation). These constraints include for example the constraint that the transformation is regular, which for example means that a Jacobian determinant calculated from a matrix of the deformation field (for example, the vector field) is larger than zero, and also the constraint that the transformed (deformed) image is not self-intersecting and for example that the transformed (deformed) image does not comprise faults and/or ruptures. The constraints include for example the constraint that if a regular grid is transformed simultaneously with the image and in a corresponding manner, the grid is not allowed to interfold at any of its locations. The optimising problem is for example solved iteratively, for example by means of an optimisation algorithm which is for example a first-order optimisation algorithm, such as a gradient descent algorithm. Other examples of optimisation algorithms include optimisation algorithms which do not use derivations, such as the downhill simplex algorithm, or algorithms which use higher-order derivatives such as Newton-like algorithms. The optimisation algorithm preferably performs a local optimisation. If there is a plurality of local optima, global algorithms such as simulated annealing or generic algorithms can be used. In the case of linear optimisation problems, the simplex method can for instance be used.

In the steps of the optimisation algorithms, the voxels are for example shifted by a magnitude in a direction such that the degree of similarity is increased. This magnitude is preferably less than a predefined limit, for instance less than one tenth or one hundredth or one thousandth of the diameter of the image, and for example about equal to or less than the distance between neighbouring voxels. Large deformations can be implemented, for example due to a high number of (iteration) steps.

The determined elastic fusion transformation can for example be used to determine a degree of similarity (or similarity measure, see above) between the first and second datasets (first and second images). To this end, the deviation between the elastic fusion transformation and an identity transformation is determined. The degree of deviation can for instance be calculated by determining the difference between the determinant of the elastic fusion transformation and the identity transformation. The higher the deviation, the lower the similarity, hence the degree of deviation can be used to determine a measure of similarity.

A measure of similarity can for example be determined on the basis of a determined correlation between the first and second datasets.

**BRIEF DESCRIPTION OF THE DRAWINGS**

In the following, the invention is described with reference to the appended figures which give background explanations and represent specific embodiments of the invention. The scope of the invention is however not limited to the specific features disclosed in the context of the figures, wherein

Fig. 1 illustrates the basic steps of the method according to the first aspect;

Fig. 2 shows an embodiment of the present invention, specifically the method according to the first aspect;

Fig. 3 is an illustration of an embodiment of the method according to the first aspect; and

Fig. 4 is a schematic illustration of the system according to the fifth aspect.

**DESCRIPTION OF EMBODIMENTS**

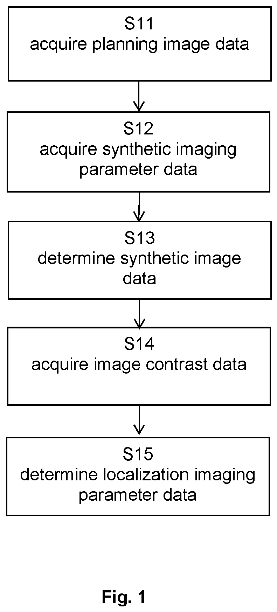

Fig. 1 illustrates the basic steps of the method according to the first aspect, in which step S11 encompasses acquisition of the planning image data, step S12 encompasses acquisition of the synthetic imaging parameter data and subsequent step S13 encompasses determination of the synthetic image data. Step S14 then acquires the image contrast data and step S15 finally determine the localization imaging parameter data.

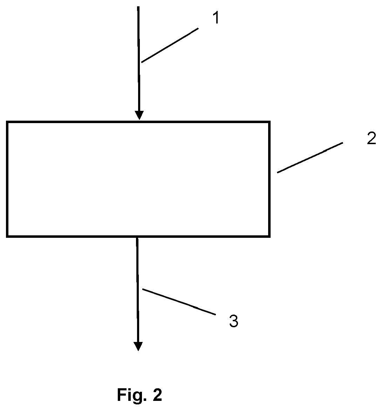

Fig. 2 illustrates an embodiment of the present invention that includes all essential features of the invention. In this embodiment, the entire data processing which is part of the method according to the first aspect is performed by a computer 2. Reference sign 1 denotes the input of data acquired by the method according to the first aspect into the computer 2 and reference sign 3 denotes the output of data determined by the method according to the first aspect.

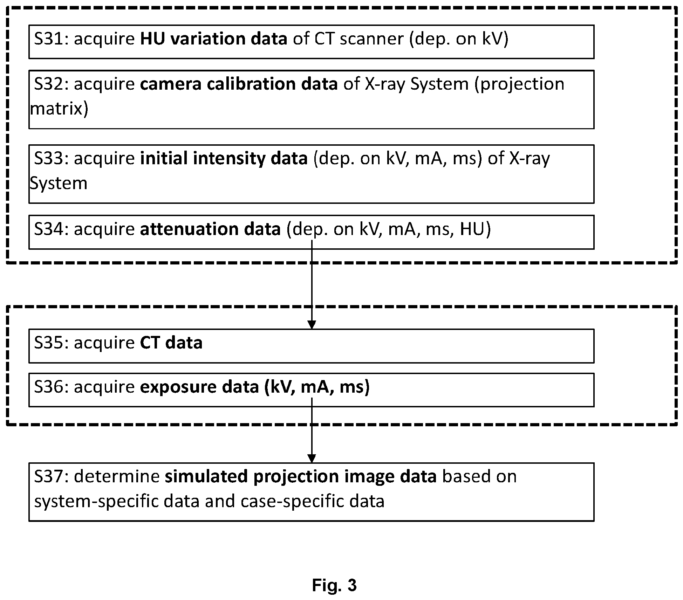

Fig. 3 is a flow diagram showing the steps of an embodiment of the method according to the first aspect. In step S31, the Hounsfield unit (HU) variation data of a CT scanner used to generate the planning image data is acquired. The Hounsfield unit variation data serves as source for the absorption coefficients (density values) for ionizing imaging radiation (such as X-rays) in the anatomical body part and the anatomical structures surrounding it. The absorption coefficients are dispersive, i.e. they depend on the voltage of the X-ray device/CT scanner used and therefore on the associated energy of the beam of imaging radiation. In step S32, camera calibration data of an X-ray system to be used for tracking the anatomical body part is acquired. Specifically, the camera calibration data defines a projection matrix and therefore the imaging geometry of the X-ray system. Step S33 then acquires the initial intensity data, i.e values for beam energy and exposure time to be used for generating the synthesized two-dimensional medical image. Attenuation data defining the attenuation of the virtual beam of imaging radiation is then determined from the previously acquired data sets in step S34. The set of parameters required for determining the DRRs from the planning image data acquired as CT data in step S3 is then acquired as exposure data in step S36, and the simulated projection image data describing the DRRs is determined in step S37 as required for the radiation treatment system and the medical case of the individual patient.

The implementation of the attenuation for determining the DRRs is based on the Beer-Lambert law: The intensity of an X-ray beam decreases exponentially with the distance which it has travelled inside a material:

$$I(z) = I0 * e^{-\mu z},$$

where:

$I0$: initial intensity I0 - without an object in line of sight

$\mu$: attenuation coefficient: depending on density (Hounsfield value (HU)) and kV values, the beam is attenuated differently (see Figure 1)

$Z$: distance travelled

Based on the known camera projection geometry, the rays creating the image are constructed. Every single ray is stepwise casted through the CT data (i.e. the planning image data), and in every step the intensity is adapted according to material (Hounsfield value) and distance traversed.

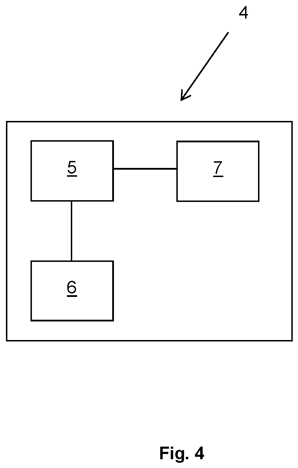

Fig. 4 is a schematic illustration of the medical system 4 according to the fifth aspect. The system is in its entirety identified by reference sign 4 and comprises a computer 5, an electronic data storage device (such as a hard disc) 6 for storing at least the patient data and a medical device 7 (such as a radiation treatment apparatus). The components of the medical system 4 have the functionalities and properties explained above with regard to the fifth aspect of this disclosure.

## Claims

1. A computer-implemented medical method of determining imaging control data for controlling medical imaging for localizing the position of an anatomical body part, the method comprising the following steps:

   a) planning image data is acquired (S11) which describes a three-dimensional digital planning medical image of the anatomical body part;
   b) synthetic imaging parameter data is acquired (S12) which describes at least one synthetic imaging parameter usable for determining a digital synthetic medical image of the anatomical body part based on the planning image data;
   c) synthetic image data is determined (S13) based on the planning image data and the synthetic imaging parameter data, wherein the synthetic image data describes a digital synthesized two-dimensional medical image of at least part of the anatomical body part;
   d) image contrast data is acquired (S14) which describes a predetermined criterion to be fulfilled by the contrast of the synthesized two-dimensional medical image;
   e) localization imaging parameter data is determined (S15) based on the synthetic imaging parameter data and the synthetic image data and the image contrast data, wherein the localization parameter data describes at least one localization imaging parameter to be used for generating a digital localization medical image of the anatomical body part for localizing the position of the anatomical body part,

   **characterized in that**
   the synthetic imaging parameter and the localization imaging parameter describe at least one of an energy of an imaging beam used for imaging the anatomical body part and an exposure time of the imaging.

2. The method according to the preceding claim, wherein the anatomical body part is subject to a changing vital state.

3. The method according to any one of the preceding claims, wherein the localization parameter data is determined if the contrast of the synthesized two-dimensional medical image fulfils the predetermined criterion.

4. The method according to any one of the preceding claims, wherein the predetermined criterion is a threshold for the contrast of the synthesized two-dimensional digital medical image.

5. The method according to any one of the two immediately preceding claims, wherein the synthetic image data is determined using the synthetic imaging parameter and the at least one localization imaging parameter corresponds to the synthetic imaging parameter.

6. The method according to any one of the preceding claims, wherein the three-dimensional digital planning medical image includes a plurality of three-dimensional digital planning medical images describing the anatomical body part in different vital states, the digital synthesized two-dimensional medical image includes a plurality of synthesized two-dimensional medical images of at least part of the anatomical body part associated with the plurality of three-dimensional digital planning medical images, and the at least one synthetic imaging parameter and the contrast of the image representation of the anatomical body part and anatomical structures surrounding the anatomical body part in the synthesized two-dimensional medical image are varied to optimize localization of the image representation the anatomical body part in each of the plurality of synthesized two-dimensional medical images.

7. The method according to any one of the preceding claims, wherein the digital synthesized two-dimensional medical image includes at least two digital synthesized two-dimensional medical images of the at least part of the anatomical body part which are or have been generated using different values of the at least one synthetic imaging parameter and the at least two digital synthesized two-dimensional medical images are weighted and subtracted from each other and the result of the subtraction is used for determining the localization imaging parameter data.

8. The method according to any one of the preceding claims, comprising

determining, based on the localization imaging parameter data, control data for controlling a medical imaging device to generate an image of the anatomical body part.

9. The method according to any one of claims 1 to 8, comprising

acquiring the parameters of a trained learning algorithm trained by inputting, to the learning algorithm, a plurality of three-dimensional planning images of the anatomical body of different patients and at least one two-dimensional radiography of the anatomical body part associated with each of the three-dimensional planning images and generated using known imaging parameters and for example having a known imaging geometry relative to the associated three-dimensional planning image,

such that the learning algorithm is trained to establish a relation between the at least one two-dimensional radiography of the anatomical body part and the synthesized two-dimensional medical images generated from the associated three-dimensional planning image,

wherein the synthetic image data is generated by the trained learning algorithm by inputting the planning image data to the trained learning algorithm.

10. A computer program comprising instructions which, when the program is executed by a computer, cause the computer to carry out the method according to any one of the preceding claims.

11. A computer readable storage medium on which the program according to the preceding claim is stored.

12. A computer comprising at least one processor configured to execute the program according to claim 10.

13. A medical system (4), comprising:

a) the computer (5) according to claim 12, wherein the instructions cause the computer to carry out the method according to claim 8;
b) at least one electronic data storage device (6) storing the control data; and
c) a radiation treatment apparatus (7) comprising at least one medical imaging device,

wherein the at least one computer is operably coupled to

- the at least one electronic data storage device for acquiring, from the at least one data storage device, the control data, and

- the radiation treatment apparatus (7) for issuing a control signal to the radiation treatment apparatus (7) for controlling the operation of the at least one medical imaging device on the basis of the control data.

**Patentansprüche**

1. Computerimplementiertes medizinisches Verfahren zum Bestimmen von Bildsteuerungsdaten zur Steuerung der medizinischen Bildgebung zur Lokalisierung der Position eines anatomischen Körperteils, wobei das Verfahren die folgenden Schritte umfasst:

a) Planungsbilddaten werden erfasst (S11), die ein dreidimensionales digitales medizinisches Planungsbild des anatomischen Körperteils beschreiben;
b) synthetische Bildparameterdaten werden erfasst (S12), die mindestens einen synthetischen Bildparameter beschreiben, der zur Bestimmung eines digitalen synthetischen medizinischen Bildes des anatomischen Körperteils auf der Grundlage der Planungsbilddaten verwendbar ist;
c) synthetische Bilddaten werden auf der Grundlage der Planungsbilddaten und der synthetischen Bildparameterdaten bestimmt (S13), wobei die synthetischen Bilddaten ein digitales synthetisiertes zweidimensionales medizinisches Bild von zumindest einem Teil des anatomischen Körperteils beschreiben;
d) Bildkontrastdaten werden erfasst (S14), die ein vorbestimmtes Kriterium beschreiben, das von dem Kontrast des synthetisierten zweidimensionalen medizinischen Bildes zu erfüllen ist;
e) Lokalisierungsabbildungsparameterdaten werden auf der Grundlage der synthetischen Abbildungsparameterdaten und der synthetischen Bilddaten und der Bildkontrastdaten bestimmt (S15), wobei die Lokalisierungsparameterdaten mindestens einen Lokalisierungsabbildungsparameter beschreiben, der zur Erzeugung eines digitalen medizinischen Lokalisierungsbildes des anatomischen Körperteils zur Lokalisierung der Position des anatomischen Körperteils verwendbar ist,

**dadurch gekennzeichnet, dass**
der synthetische Bildgebungsparameter und der Lokalisierungsbildgebungsparameter mindestens eine Energie eines Bildgebungsstrahls, der zur Bildgebung des anatomischen Körperteils verwendet wird, und eine Belichtungszeit der Bildgebung beschreiben.

2. Verfahren nach dem vorhergehenden Anspruch, wobei das anatomische Körperteil einem sich ändernden Vitalzustand unterworfen ist.

3. Verfahren nach einem der vorhergehenden Ansprüche, wobei die Lokalisierungsparameterdaten ermittelt werden, wenn der Kontrast des synthetisierten zweidimensionalen medizinischen Bildes das vorgegebene Kriterium erfüllt.

4. Verfahren nach einem der vorangehenden Ansprüche, wobei das vorgegebene Kriterium ein Schwellenwert für den Kontrast des synthetisierten zweidimensionalen medizinischen Bildes ist.

5. Verfahren nach einem der beiden vorhergehenden Ansprüche, wobei die synthetischen Bilddaten unter Verwendung des synthetischen Abbildungsparameters bestimmt werden und der mindestens eine Lokalisationsabbildungsparameter dem synthetischen Abbildungsparameter entspricht.

6. Verfahren nach einem der vorhergehenden Ansprüche, wobei das dreidimensionale digitale medizinische Planungsbild eine Vielzahl von dreidimensionalen digitalen medizinischen Planungsbildern enthält, die den anatomischen Körperteil in verschiedenen Vitalzuständen beschreiben, das digitale synthetisierte zweidimensionale medizinische Bild eine Vielzahl von synthetisierten zweidimensionalen medizinischen Bildern von mindestens einem Teil des anatomischen Körperteils enthält, die mit der Vielzahl von dreidimensionalen digitalen medizinischen Planungsbildern verbunden sind, und der mindestens eine synthetische Bildgebungsparameter und der Kontrast der Bilddarstellung des anatomischen Körperteils und der anatomischen Strukturen, die den anatomischen Körperteil in dem synthetisierten zweidimensionalen medizinischen Bild umgeben, variiert werden, um die Lokalisierung der Bilddarstellung des anatomischen Körperteils in jedem der Vielzahl von synthetisierten zweidimensionalen medizinischen Bildern zu optimieren.

7. Verfahren nach einem der vorhergehenden Ansprüche, wobei das digitale synthetisierte zweidimensionale medizinische Bild mindestens zwei digitale synthetisierte zweidimensionale medizinische Bilder des mindestens einen Teils des anatomischen Körperteils enthält, die unter Verwendung unterschiedlicher Werte des mindestens einen synthetischen Bildgebungsparameters erzeugt werden oder worden sind, und die mindestens zwei digitalen synthetisierten zweidimensionalen medizinischen Bilder gewichtet und voneinander subtrahiert werden und das Ergebnis der Subtraktion zur Bestimmung der Lokalisierungsbildparameterdaten verwendet wird.

8. Verfahren nach einem der vorhergehenden Ansprüche, umfassend
Bestimmen, auf der Grundlage der Lokalisierungsbildparameterdaten, von Steuerdaten zur Steuerung einer medizinischen Bildgebungsvorrichtung zur Erzeugung eines Bildes des anatomischen Körperteils.

9. Verfahren nach einem der Ansprüche 1 bis 8, umfassend

Erfassen der Parameter eines trainierten Lernalgorithmus, der dadurch trainiert wird, dass in den Lernalgorithmus mehrere dreidimensionale Planungsbilder des anatomischen Körpers verschiedener Patienten und mindestens eine zweidimensionale Röntgenaufnahme des anatomischen Körperteils eingegeben werden, die jedem der dreidimensionalen Planungsbilder zugeordnet ist und unter Verwendung bekannter Bildgebungsparameter erzeugt wird und beispielsweise eine bekannte Bildgebungsgeometrie in Bezug auf das zugeordnete dreidimensionale Planungsbild aufweist,
so dass der Lernalgorithmus trainiert wird, um eine Beziehung zwischen der mindestens einen zweidimensionalen Röntgenaufnahme des anatomischen Körperteils und den synthetisierten zweidimensionalen medizinischen Bildern herzustellen, die aus dem zugehörigen dreidimensionalen Planungsbild erzeugt werden, wobei die synthetischen Bilddaten durch den trainierten Lernalgorithmus erzeugt werden, indem die Planungsbilddaten in den trainierten Lernalgorithmus eingegeben werden.

10. Computerprogramm mit Anweisungen, die, wenn das Programm von einem Computer ausgeführt wird, den Computer veranlassen, das Verfahren nach einem der vorhergehenden Ansprüche auszuführen.

11. Computerlesbares Speichermedium, auf dem das Programm nach dem vorhergehenden Anspruch gespeichert ist.

12. Computer mit mindestens einem Prozessor, der zur Ausführung des Programms nach Anspruch 10 konfiguriert ist.

13. Medizinisches System (4), umfassend:

a) den Computer (5) nach Anspruch 12, wobei die Anweisungen den Computer veranlassen, das Verfahren nach Anspruch 8 auszuführen;
b) mindestens eine elektronische Datenspeichereinrichtung (6), die die Steuerdaten speichert; und

c) ein Strahlentherapiegerät (7), das mindestens eine medizinische Bildgebungsvorrichtung umfasst,

wobei der mindestens eine Computer betriebsfähig gekoppelt ist mit

- mit der mindestens einen elektronischen Datenspeichervorrichtung verbunden ist, um von der mindestens einen Datenspeichervorrichtung die Steuerdaten zu erfassen, und
- das Strahlenbehandlungsgerät (7) zum Ausgeben eines Steuersignals an das Strahlentherapiegerät (7) zum Steuern des Betriebs der mindestens einen medizinischen Bildgebungsvorrichtung auf der Grundlage der Steuerdaten.

## Revendications

1. Procédé médical mis en œuvre par ordinateur pour déterminer des données de commande d'imagerie afin de commander une imagerie médicale pour localiser la position d'une partie corporelle anatomique, le procédé comprenant les étapes suivantes :

a) des données d'image de planification sont acquises (S11) qui décrivent une image médicale de planification numérique tridimensionnelle de la partie corporelle anatomique ;
b) des données de paramètres d'imagerie synthétique sont acquises (S12) qui décrivent au moins un paramètre d'imagerie synthétique utilisable pour déterminer une image médicale synthétique numérique de la partie corporelle anatomique sur la base des données d'image de planification ;
c) des données d'image synthétique sont déterminées (S13) sur la base des données d'images de planification et des données de paramètres d'imagerie synthétique, dans lequel les données d'image synthétique décrivent une image médicale bidimensionnelle synthétisée numérique d'au moins une partie de la partie corporelle anatomique ;
d) des données de contraste d'image sont acquises (S14) qui décrivent un critère prédéterminé auquel doit répondre le contraste de l'image médicale bidimensionnelle synthétisée ;
e) des données de paramètre d'imagerie de localisation sont déterminées (S15) sur la base des données de paramètre d'imagerie synthétique et des données d'image synthétique et des données de contraste d'image, dans lequel les données de paramètre de localisation décrivent au moins un paramètre d'imagerie de localisation à utiliser pour générer une image médicale numérique de localisation de la partie corporelle anatomique afin de localiser la position de la partie corporelle anatomique,

**caractérisé en ce que**
le paramètre d'imagerie synthétique et le paramètre d'imagerie de localisation décrivent au moins l'un parmi une énergie d'un faisceau d'imagerie utilisé pour imager la partie corporelle anatomique et un temps d'exposition de l'imagerie.

2. Procédé selon la revendication précédente, dans lequel la partie corporelle anatomique est soumise à un état vital changeant.

3. Procédé selon l'une quelconque des revendications précédentes, dans lequel les données de paramètre de localisation sont déterminées si le contraste de l'image médicale bidimensionnelle synthétisée répond au critère prédéterminé.

4. Procédé selon l'une quelconque des revendications précédentes, dans lequel le critère prédéterminé est un seuil pour le contraste de l'image médicale numérique bidimensionnelle synthétisée.

5. Procédé selon l'une quelconque des deux revendications immédiatement précédentes, dans lequel les données d'image synthétique sont déterminées à l'aide du paramètre d'imagerie synthétique et l'au moins un paramètre d'imagerie de localisation correspond au paramètre d'imagerie synthétique.

6. Procédé selon l'une quelconque des revendications précédentes, dans lequel l'image médicale de planification numérique tridimensionnelle inclut une pluralité d'images médicales de planification numériques tridimensionnelles décrivant la partie corporelle anatomique dans différents états vitaux, l'image médicale bidimensionnelle synthétisée numérique inclut une pluralité d'images médicales bidimensionnelles synthétisées d'au moins une partie de la partie corporelle anatomique associée à la pluralité d'images médicales de planification numériques tridimensionnelles, et l'au moins un paramètre d'imagerie synthétique et le contraste de la représentation d'image de la partie corporelle anatomique et des structures anatomiques entourant la partie corporelle anatomique dans l'image médicale bidimensionnelle synthétisée sont modifiés pour optimiser une localisation de la représentation d'image de la partie corporelle anatomique dans chacune de la pluralité d'images médicales bidimensionnelles synthétisées.

7. Procédé selon l'une quelconque des revendications précédentes, dans lequel l'image médicale bidimensionnelle synthétisée numérique inclut au moins deux images médicales bidimensionnelles synthétisées numériques de l'au moins une partie de la partie corporelle anatomique qui sont ou ont été générées à l'aide de différentes valeurs de l'au moins un paramètre d'imagerie synthétique et les au moins deux images médicales bidimensionnelles synthétisées numériques sont pondérées et soustraites l'une de l'autre et le résultat de la soustraction est utilisé pour déterminer les données de paramètre d'imagerie de localisation.

8. Procédé selon l'une quelconque des revendications précédentes, comprenant
la détermination, sur la base des données de paramètre d'imagerie de localisation, de données de commande pour commander un dispositif d'imagerie médicale afin de générer une image de la partie corporelle anatomique.

9. Procédé selon l'une quelconque des revendications 1 à 8, comprenant

l'acquisition des paramètres d'un algorithme d'apprentissage entraîné qui est entraîné par la fourniture en entrée, à l'algorithme d'apprentissage, d'une pluralité d'images de planification tridimensionnelles du corps anatomique de différents patients et d'au moins une radiographie bidimensionnelle de la partie corporelle anatomique associée à chacune des images de planification tridimensionnelle et générée à l'aide de paramètres d'imagerie connus et, par exemple, présentant une géométrie d'imagerie connue par rapport à l'image de planification tridimensionnelle associée,
de sorte que l'algorithme d'apprentissage est entraîné à établir une relation entre l'au moins une radiographie bidimensionnelle de la partie corporelle anatomique et les images médicales bidimensionnelles synthétisées générées à partir de l'image de planification tridimensionnelle associée,
dans lequel les données d'image synthétiques sont générées par l'algorithme d'apprentissage entraîné par fourniture en entrée des données d'image de planification à l'algorithme d'apprentissage entraîné.

10. Programme informatique comprenant des instructions qui, lorsque le programme est exécuté par un ordinateur, amènent l'ordinateur à mettre en œuvre le procédé selon l'une quelconque des revendications précédentes.

11. Support de stockage lisible par ordinateur sur lequel le programme selon la revendication précédente est stocké.

12. Ordinateur comprenant au moins un processeur configuré pour exécuter le programme selon la revendication 10.

13. Système médical (4), comprenant :

a) l'ordinateur (15) selon la revendication 12, dans lequel les instructions amènent l'ordinateur à mettre en œuvre le procédé selon la revendication 8 ;
b) au moins un dispositif électronique de stockage de données (6) stockant les données de commande ; et
c) un appareil de radiothérapie (7) comprenant au moins un dispositif d'imagerie médicale,
dans lequel l'au moins un ordinateur est couplé de manière opérationnelle

- à l'au moins un dispositif électronique de stockage de données pour acquérir, à partir de l'au moins un dispositif de stockage de données, les données de commande, et
- l'appareil de radiothérapie (7) pour émettre un signal de commande à l'appareil de radiothérapie (7) afin de commander le fonctionnement de l'au moins un dispositif d'imagerie médicale sur la base des données de commande.

S11
acquire planning image data

↓

S12
acquire synthetic imaging
parameter data

↓

S13
determine synthetic image
data

↓

S14
acquire image contrast data

↓

S15
determine localization imaging
parameter data

**Fig. 1**

**Fig. 2**

S31: acquire **HU variation data** of CT scanner (dep. on kV)

S32: acquire **camera calibration data** of X-ray System (projection matrix)

S33: acquire **initial intensity data** (dep. on kV, mA, ms) of X-ray System

S34: acquire **attenuation data** (dep. on kV, mA, ms, HU)

S35: acquire **CT data**

S36: acquire **exposure data (kV, mA, ms)**

S37: determine **simulated projection image data** based on system-specific data and case-specific data

**Fig. 3**

4

5

7

6

**Fig. 4**

# EP 4 522 267 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20220305290 A1
- EP 08156293 A
- US 61054187 B

### Non-patent literature cited in the description

- **ROGER Y. TSAI**. An Efficient and Accurate Camera Calibration Technique for 3D Machine Vision. *Proceedings of the IEEE Conference on Computer Vision and Pattern Recognition*, 1986, 364-374
- **ROGER Y. TSAI**. A Versatile Camera Calibration Technique for High-Accuracy 3D Machine Vision Metrology Using Off-the-Shelf TV Cameras and Lenses. *IEEE Journal of Robotics and Automation*, August 1987, vol. RA-3 (4), 323-344
- Fluoroscopic image processing for computer-aided orthopaedic surgery. **YANIV Z.** ; **JOSKOWICZ L.** ; **SIMKIN A.** ; **GARZA-JINICH M.** ; **MILGROM C.** Medical Image Computing and Computer-Assisted Intervention - MICCAI'98. MICCAI 1998. Springer, 1998, vol. 1496